# EUROPEAN PATENT APPLICATION

(11) **EP 3 424 413 A1**
(43) Date of publication of application: **09.01.2019**
(21) Application number: 18181542.4
(22) Date of filing: 03.07.2018
(51) Int. Cl.: A61B 5/00, A61B 5/145

(54) **MEASURING APPARATUS AND DISPLAY APPARATUS**

(30) Priority: 04.07.2017 JP 2017131325; 28.06.2018 JP 2018123610
(71) Applicant: ARKRAY, Inc., Kyoto-shi, Kyoto 601-8045 (JP)
(72) Inventor: SHIMIZU, Takeshi, Kyoto-shi, Kyoto 602-0008 (JP)
(74) Representative: MacDougall, Alan John Shaw

(57) **Abstract**

A measuring apparatus performs wireless communications with a display apparatus, the display apparatus displaying a measurement result of a specified substance in a body fluid. The measuring apparatus includes a sensor, a transmitting unit and a touch sensor. The sensor is configured to be indwelled in vivo. The transmitting unit is configured to transmit data relating to the specified substance obtained by using the sensor. The touch sensor is configured to detect a touch operation of a user. The transmitting unit transmits a connection request to the display apparatus when the touch sensor detects the touch operation.

## Description

### FIELD

The present disclosure relates to a measuring apparatus and a display apparatus.

### BACKGROUND

There is a mesuring system that enables a single display apparatus to connect with a plurality of measuring apparatuses. Each measuring apparatus includes a sensor indwelled in vivo of a measurement examinee, and consecutively measures a specified substance in vivo by using this sensor. A measurement result is transmitted to the display apparatus at a proper timing. The display apparatus displays the measurement result received from each measuring apparatus (refer to, e.g., Non-Patent Document 1).

### [Document of Related Art]

### [Non-Patent Document]

[Non-Patent Document 1] "Freestyle Libre Pro Catalogue", issued on March 8, 2017, by Abbott Japan Co., Ltd., Japan

### SUMMARY

In the related art, the transmission of the measurement result to the display apparatus from each measuring apparatus involves using Near Field radio Communication (NFC). According to the NFC, the measuring apparatus is made to approach the display apparatus or is brought into contact therewith, whereby the measurement result is transmitted to the display apparatus from the measuring apparatus. However, the measuring apparatus includes the sensor indwelled in vivo of the measurement examinee, and it is therefore difficult to cause the measuring apparatus to approach the display apparatus, e.g., in such a case that the measurement examinee is kept sleeping, which leads to a difficulty of obtaining the measurement result in real time as the case may be.

The single display apparatus is shared between or among the plurality of measuring apparatuses, and hence such a case arises that the measurement results are mistaken for each other. "Being mistaken" occurs due to, e.g., mis-recognition of the measuring apparatus that is a source apparatus of transmitting the measurement result as the case may be.

One aspect of a technology of the disclosure aims at providing a measuring apparatus enabling a connection between a display apparatus and a desired measuring apparatus to be easily established, and providing the display apparatus.

One aspect of a technology of the disclosure is exemplified by a measuring apparatus given below. The measuring apparatus performs wireless communications with a display apparatus, the display apparatus displaying a measurement result of a specified substance in a body fluid. The measuring apparatus includes a sensor, a transmitting unit and a touch sensor. The sensor is configured to be indwelled in vivo. The transmitting unit is configured to transmit data relating to the specified substance obtained by using the sensor. The touch sensor is configured to detect a touch operation of a user. The transmitting unit transmits a connection request to the display apparatus when the touch sensor detects the touch operation.

The present measuring apparatus enables the connection between the display apparatus and the desired measuring apparatus to be easily established.

### BRIEF DESCRIPTION OF THE DRAWINGS

FIG. 1 is a view illustrating one example of a configuration of a measuring system according to a first embodiment;
FIG. 2 is a diagram illustrating one example of a configuration of a measuring apparatus according to the first embodiment;
FIG. 3 is a diagram illustrating one example of a configuration of a display apparatus according to the first embodiment;
FIG. 4 is a diagram illustrating one example of the display apparatus including a display on which a message prompting "pairing" is displayed;
FIG. 5 is a diagram illustrating one example of an external appearance of a detection apparatus according to the first embodiment;
FIG. 6 is a diagram illustrating one example of a configuration of the detection apparatus according to the first embodiment;
FIG. 7 is a flowchart illustrating one example of a processing flow of "pairing" according to the first embodiment;
FIG. 8 is a diagram illustrating one example of a configuration of the display apparatus according to a first modified example;
FIG. 9 is a flowchart illustrating one example of a processing flow of "pairing" according to the first modified example;
FIG. 10 is a diagram illustrating one example of the display apparatus including the display on which information representing the measuring apparatus is displayed;
FIG. 11 is a diagram illustrating one example of the processing flow of "pairing" according to a second modified example;
FIG. 12 is a diagram illustrating one example of a display apparatus in which to display a message prompting "pairing" for associating the measuring apparatus with the detection apparatus;
FIG. 13 is a diagram illustrating one example of a table configured to associate identifying information of the measuring apparatus with identifying information of the detection apparatus;
FIG. 14 is a view illustrating a configuration of a measuring system according to a second embodiment;
FIG. 15 is a block diagram illustrating a configuration of a dosage apparatus according to the second embodiment;
FIG. 16 is a flowchart illustrating one example of a processing flow of "pairing" according to the second embodiment; and
FIG. 17 is a diagram illustrating one example of a table configured to associate identifying information of the measuring apparatus with identifying information of the dosage apparatus.

### DESCRIPTION OF EMBODIMENTS

### <Embodiment>

An embodiment of the present invention will hereinafter be described with reference to the drawings. A configuration of the following embodiment is an exemplification, and the present invention is not limited to the configuration of the embodiment.

### <First Embodiment

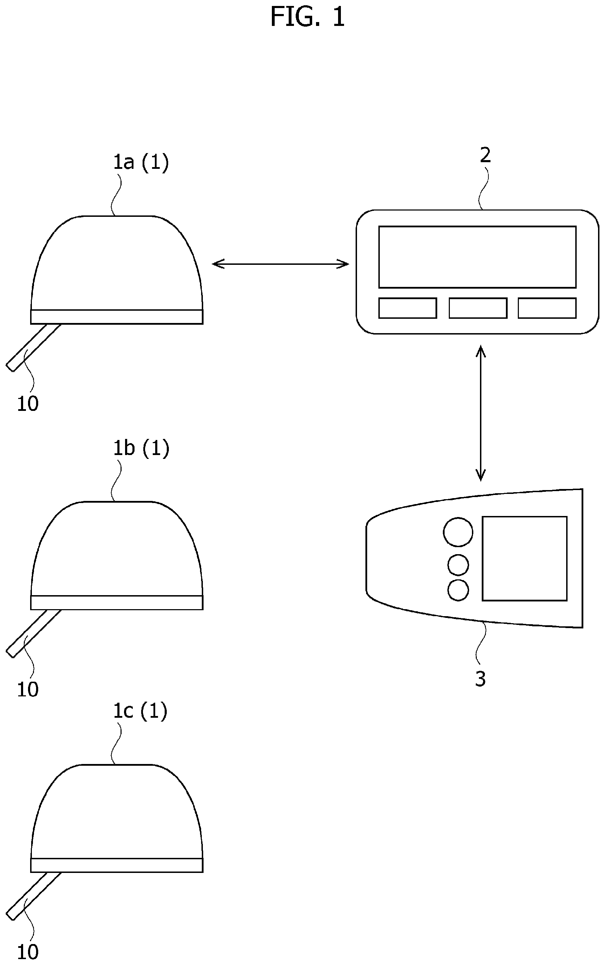

FIG. 1 is a view illustrating one example of a configuration of a measuring system according to the first embodiment. The measuring system illustrated in FIG. 1 includes a plurality of measuring apparatuses 1a, 1b, 1c, a display apparatus 2, and a detection apparatus 3. The measuring apparatuses 1a, 1b, 1c will be generically termed a measuring apparatus(es) 1. The measuring apparatus 1 is an apparatus configured to consecutively measure concentrations of specified substances (measurement target substances) of in-vivo body fluids of human bodies and animals. The measuring apparatus 1 is usable in a state of being attached to a region instanced by an abdominal region and a shoulder of a measurement examinee. The measuring apparatus 1 may also be a Continuous Glucose Monitoring (CGM) apparatus for performing continuous glucose monitoring. The in-vivo body fluid is, e.g., the body fluid instanced by an interstitial fluid. The specified substance is instanced by glucose contained in the interstitial fluid. The specified substances may also be substances other than glucose. The measuring apparatuses 1a, 1b, 1c make measurements targeted at the measurement examinees different from each other.

Communications are performed as wireless communications using radio waves, infrared rays and other equivalent radiations between the display apparatus 2 and the measuring apparatus 1, and between the display apparatus 2 and the detection apparatus 3. The wireless communications according to the first embodiment involve setting connections between the apparatuses performing the communications with each other. The setting such as this is also referred to as "pairing". It is also referred to as "establishment of paring" to establish the connection. The establishment of pairing between the apparatuses enables data to be transmitted and received therebetween. A communication method of the wireless communications is, e.g., Bluetooth (registered trademark). The establishment of pairing is done based on, e.g., Link Manager Protocol (LMP). Note that the detection apparatus 3 may also perform the data communications with the display apparatus 2 using cable communications via a communication cable.

The detection apparatus 3 is an apparatus that measures the concentration of the specified substance in the body fluid sampled from in vivo. The body fluid sampled from in vivo is, e.g., a body fluid instanced by blood. The detection apparatus 3 may also be a Self-Monitoring of Blood Glucose (SMBG) apparatus for conducting the SMBG. The detection apparatus 3 monitors a current value corresponding to a glucose concentration in the blood. The detection apparatus 3 converts the current value into a glucose concentration value in the blood with reference to calibration curve data, thereby measuring the glucose concentration value in the blood. The current value and the glucose concentration value in the blood, which are measured by the detection apparatus 3, are transmitted and inputted to the display apparatus 2. The detection apparatus 3 may also display the glucose concentration value in the blood on a display provided on the detection apparatus 3. The measurement examinee or a medical doctor or an equivalent person examining the measurement examinee may input, to the display apparatus 2, the glucose concentration value in the blood which is displayed on the display of the detection apparatus 3.

### <Measuring Apparatus 1>

The measuring apparatus 1 includes a sensor 10 used in a state of indwelled subcutaneously in the measurement examinee. The measuring apparatus 1 is attached to the measurement examinee by being pasted to a skin of the measurement examinee with an adhesive tape and other equivalent adhesives, or being fitted thereto with a belt and other equivalent fittings. The sensor 10 is an electrochemical sensor to detect the specified substance in the sample by utilizing electrochemical reaction. The sensor 10 is subcutaneously indwelled over a continuos measurement period as long as, e.g., several days through several weeks, and the measuring apparatus 1 thus consecutively measures the glucose concentration in the interstitial fluid. "Being consecutive" connotes that the measuring apparatus 1 continuously measures the glucose concentration in a state of the sensor 10 being subcutaneously indwelled, and includes such a mode that the measuring apparatus 1 measures the glucose concentration at an interval of predetermined time. A measurement frequency of the glucose concentration may be arbitrarily set with respect to the measuring apparatus 1. For example, the measurement frequency of the glucose concentration may also be set with respect to the measuring apparatus 1 so that the measuring apparatus 1 measures the glucose concentration at a frequency of once every several ten seconds through several minutes.

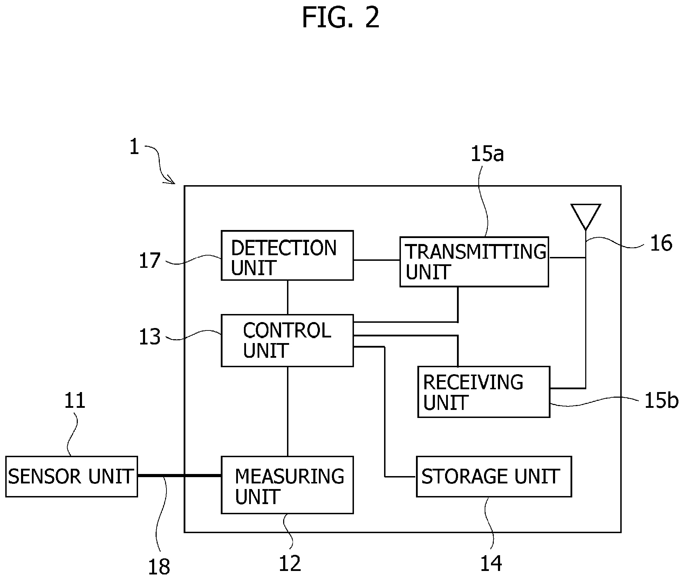

FIG. 2 is a diagram illustrating one example of a configuration of the measuring apparatus 1 according to the first embodiment. The measuring apparatus 1 is a transmitter that transmits various items of data to the display apparatus 2. The measuring apparatus 1 includes a sensor unit 11, a measurement unit 12, a control unit (arithmetic unit) 13, a storage unit 14, a transmitting unit 15a, a receiving unit 15b, an antenna 16, and a detection unit 17. The sensor unit 11 has glucose oxidoreductase instanced by glucose oxidase (GOD) and glucose dehydrogenase (GDH), and a plurality of electrodes, i.e., a working electrode, a counter electrode, a reference electrode and other equivalent electrodes. The sensor unit 11 is provided on the side of a tip of the sensor 10 illustrated in FIG. 1. The sensor 10 is electrically connected to the measurement unit 12 via a wire 18.

The measurement unit 12 is a circuit to measure a signal value (e.g., a response current value) by applying a voltage to the sensor unit 11. When the voltage is applied to between the electrodes (between the working electrode and the counter electrode, or between the working electrode and the reference electrode) of the sensor unit 11, the sensor unit 11 outputs a response current value corresponding to the glucose concentration in the body fluid. The measurement unit 12 measures the response current value outputted from the sensor unit 11 in a way that controls the voltage to be applied to between the electrodes of the sensor unit 11. When the voltage is applied to between the electrodes of the sensor unit 11, the glucose in the body fluid is oxidized by the oxidoreductase, and electrons being thereby extracted are supplied to the working electrode. The measurement unit 12 measures, as the response current value, a quantity of electric charges of the electrons supplied to the working electrode. The measurement unit 12 converts the response current value into a response voltage value, and may measure, as the response voltage value, the quantity of electric charges of the electrons supplied to the working electrode. The following discussion will deal with a case that the measurement unit 12 measures the response current value. The measurement unit 12 sends the measured response current value to the control unit 13. The response current value and the response voltage value are each one example of "data relating to the specified substance".

The control unit 13 controls the measurement unit 12, the storage unit 14, the transmitting unit 15a, the receiving unit 15b and the detection unit 17. The control unit 13, the storage unit 14, the transmitting unit 15a and the receiving unit 15b may be attained by: computers each including a Central Processing Unit (CPU), a Random Access Memory (RAM), a Read Only Memory (ROM) and other equivalent hardware components that are provided in the measuring apparatus 1; respective apparatuses; and programs and other equivalent software components running on the computer. The CPU is also called a processor. It does not mean that the CPU is limited to the single processor, and the CPU may, however, take a multi-processor configuration.

The control unit 13 stores the response current value in the storage unit 14, and sends the response current value to the transmitting unit 15a. The transmitting unit 15a transmits the response current value to the display apparatus 2 via the antenna 16. The transmitting unit 15a may receive the response current value from the control unit 13, and may also acquire the response current value from the storage unit 14. The receiving unit 15b receives the various items of data from the display apparatus 2 via the antenna 16, and notifies the control unit 13 of the received data.

The detection unit 17 is a sensor to detect a touch operation done by a user instanced by the measurement examinee and the medical doctor. The touch operation is exemplified by a tap operation that the user taps the measuring apparatus 1 with a finger and other equivalent regions and a swipe operation that the finger or another equivalent region is moved so as to sweep on the measuring apparatus 1. There is no particular limit to a method by which the detection unit 17 detects the touch operation. The detection unit 17 may also be, e.g., a contact type touch panel. The method, by which the touch panel detects the touch operation, may be exemplified by an electrostatic capacitance method, a piezoelectric method, a resistive film method, and a surface acoustic wave (SAW) method. The detection unit 17, which may be an acceleration sensor, may detect the touch operation by detecting acceleration generated on the measuring apparatus 1 upon performing the touch operation. The detection unit 17 may be a coordinate sensor, and may detect the touch operation by detecting coordinates of a position in which an external force is applied to the measuring apparatus 1 through the touch operation. The detection unit 17, upon detecting the touch operation, notifies the transmitting unit 15a of a purport that the touch operation has been detected. The detection unit 17 notifies the transmitting unit 15a of the purport that the touch operation has been detected, by transmitting, e.g., pulse waves to the transmitting unit 15a. The detection unit 17 is one example of a "touch sensor".

The transmitting unit 15a transmits, to the display apparatus 2, the response current value received from the control unit 13. The transmitting unit 15a transmits, as triggered by an event that the detection unit 17 has detected the touch operation, a connection request for establishing "pairing" to the display apparatus 2 via the antenna 16. The connection request contains, e.g., identifying information of the measuring apparatus 1. The identifying information of the measuring apparatus 1 is, for instance, Bluetooth (registered trademark) Device Address (BD Address) of the measuring apparatus 1.

### <Display apparatus 2>

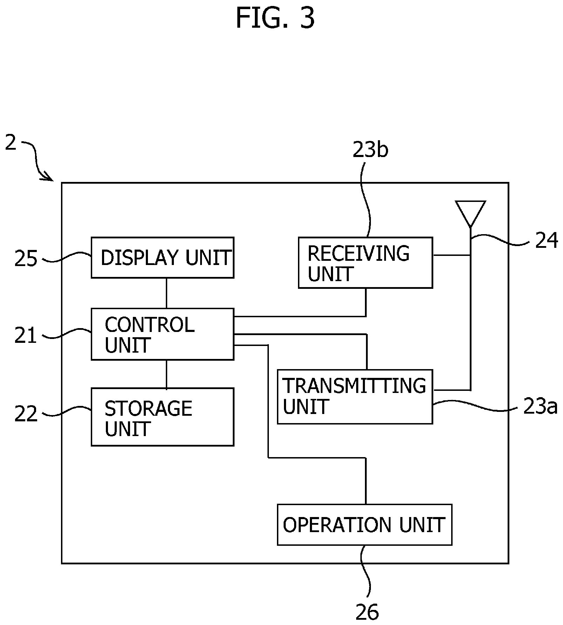

FIG. 3 is a diagram illustrating one example of a configuration of the display apparatus 2 according to the first embodiment. The display apparatus 2 receives the various items of data from the measuring apparatus 1, and displays the received data. The display apparatus 2 includes a control unit (arithmetic unit) 21, a storage unit 22, a transmitting unit 23a, a receiving unit 23b, an antenna 24, a display unit 25, and an operation unit 26. The control unit 21 controls the storage unit 22, the transmitting unit 23a, the receiving unit 23b, the display unit 25, and the operation unit 26. The control unit 21, the storage unit 22, the transmitting unit 23a and the receiving unit 23b may be attained by: computers each including the CPU, the RAM, the ROM and other equivalent hardware components that are provided in the display apparatus 2; respective apparatuses; and programs and other equivalent software components running on the computer.

The display unit 25 has a display and displays various types of information and messages on this display. For example, a message prompting "pairing" with the measuring apparatus 1 is displayed on the display unit 25. The display unit 25 displays a measurement result and an error on the display, and also displays operation procedures, operation statuses and other equivalent items when setting is done. The display of the display unit 25 is exemplified by a liquid crystal display apparatus, a plasma display panel, a Cathode Ray Tube (CRT) display, or an Electroluminescence (EL) panel. The operation unit 26 includes a variety of operation buttons, a touch panel and other equivalent components, and accepts a user's operation.

The control unit 21 receives the connection request from the measuring apparatus 1 or the detection apparatus 3 via the antenna 24 and the receiving unit 23b. The control unit 21 establishes "pairing" with the measuring apparatus 1 or the detection apparatus 3, which is a transmission source of the connection request. When establishing "pairing", the identifying information contained in the connection request may be stored in the storage unit 22. The control unit 21 accepts the data from the measuring apparatus 1 or the detection apparatus 3 established "pairing". The control unit 21 stores the received response current value in the storage unit 22. Calibration curve data indicating an associative relation between the response current value and the glucose concentration in the interstitial fluid is pre-stored in the storage unit 22. The calibration curve data pre-stored in the storage unit 22 may be a mathematical expression for calculating the glucose concentration from the response current value. The calibration curve data pre-stored in the storage unit 22 may also be an associative table representing the associative relation between the response current value and the glucose concentration. The control unit 21 converts the response current value into the glucose concentration with reference to the calibration curve data stored in the storage unit 22. The control unit 21 functions as a calculation unit to calculate the glucose concentration in the interstitial fluid, based on the response current value. The control unit 21 outputs the accepted data to the display unit 25. The control unit 21 may determine whether the measuring apparatus 1 or the detection apparatus 3 is established "paring", based on whether identifying information coincident with the identifying information of the measuring apparatus 1 or the detection apparatus 3 having transmitted the data is pre-installed in the storage unit 22.

The transmitting unit 23a transmits the various items of data via the antenna 24. The transmitting unit 23a transmits the connection request to, e.g., the measuring apparatus 1. The connection request contains the identifying information of, e.g., the display apparatus 2. The identifying information of the display apparatus 2 is, for example, the BD Address of the display apparatus 2.

The receiving unit 23b receives the various items of data via the antenna 24. The receiving unit 23b, upon receiving the connection request from, e.g., the measuring apparatus 1 or the detection apparatus 3, sends the received connection request to the control unit 21. The receiving unit 23b, upon further receiving the response current value from the measuring apparatus 1, sends the received response current value to the control unit 21.

The receiving unit 23b receives a reference value related to the glucose in the blood (which will hereinafter be simply referred to as the reference value) from the detection apparatus 3 via the antenna 24, and sends the reference value to the control unit 21. The control unit 21 stores, in the storage unit 22, the reference value received from the detection apparatus 3 established "pairing". The control unit 21 corrects the glucose concentration in the interstitial fluid by using the reference value. The glucose concentration value in the blood is not coincident with the glucose concentration value in the interstitial fluid. The control unit 21 therefore executes a process of getting the glucose concentration value in the interstitial fluid approximate to the glucose concentration value in the blood by correcting the glucose concentration value in the interstitial fluid in a way that uses the reference value. The reference value is one example of "data for correction".

The glucose reference value is, e.g., the current value measured by the detection apparatus 3 or the glucose concentration value in the blood, which is measured by the detection apparatus 3. The control unit 21 calculates, based on the response current value, the glucose concentration value in the interstitial fluid, and may correct the glucose concentration value in the interstitial fluid with reference to the glucose concentration value in the blood which is measured by the detection apparatus 3. The control unit 21 corrects the response current value with reference to the current value measured by the detection apparatus 3, and may calculate the glucose concentration value in the interstitial fluid on the basis of the post-correcting response current value.

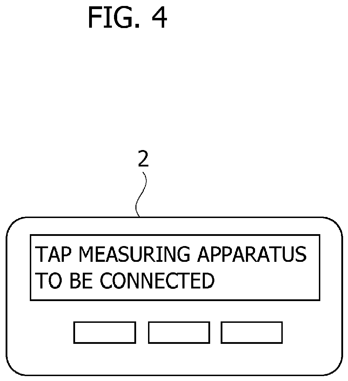

The control unit 21 causes the display unit 25 to display the message prompting to establish "pairing" with the measuring apparatus 1, for example, in response to the operation on the operation unit 26. FIG. 4 is a diagram illustrating one example of the display apparatus 2, with the message prompting "pairing" being displayed on its display. The control unit 21, upon displaying the message prompting "pairing" on its display, transitions to a waiting status of accepting the connection request. The control unit 21 transitioning to the waiting status, when receiving the connection request from the measuring apparatus 1 via the receiving unit 23b, establishes "pairing" with the measuring apparatus 1 which is the transmission source of the connection request. For example, the control unit 21 may store the identifying information contained in the connection request in the storage unit 22 when establishing "pairing". The receiving unit 23b sends, to the control unit 21, the response current value transmitted from the measuring apparatus 1 established "pairing". It may be sufficient that time of duration of the waiting status is properly determined. Note that the control unit 21 not transitioning to the waiting status discards the connection request received via the receiving unit 23b.

### <Detection Apparatus 3>

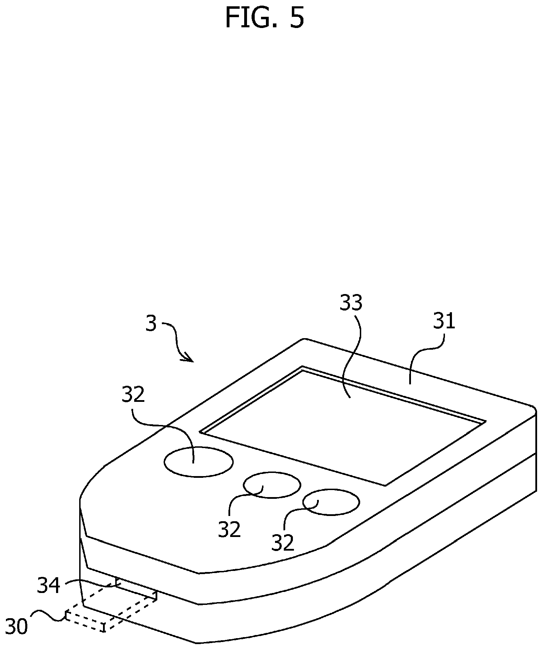

FIG. 5 is a view illustrating one example of an external appearance of the detection apparatus 3 according to the first embodiment. The detection apparatus 3 measures the glucose concentration in the blood by an electrochemical method using a biosensor 30. The detection apparatus 3 includes a housing 31, a plurality of operation buttons 32, a display panel 33, and a sensor insertion port 34.

As illustrated in FIG. 5, the housing 31 is provided with the operation buttons 32 and the display panel 33. The operation buttons 32 are used for making various settings (setting of measurement conditions, inputting of user's ID and other equivalent settings), and for conducting operations to start and finish the measurement. The operation buttons 32 may also be a contact type touch panel. The display panel 33 displays the measurement result and the error, and further displays the operation procedures, the operation statuses and other equivalent items when setting is done. The display panel 33 is exemplified by the liquid crystal display apparatus, the plasma display panel, the CRT display, or the Electroluminescence panel. The contact type touch panel is disposed by being superposed on the display panel 33, whereby the operation buttons 32 may be integral with the display panel 33.

The biosensor 30 includes a substrate, a plurality of electrodes, i.e., the working electrode, the counter electrode and the reference electrode each provided on the substrate, and the glucose oxidoreductase. A capillary is formed inside of the biosensor 30. The capillary of the biosensor 30 is provided with a reagent layer, and retains the blood. The biosensor 30 is inserted into the sensor insertion port 34. The detection apparatus 3 applies a voltage to the biosensor 30, and thus measures a signal value (e.g., the current value). When the voltage is applied to between the electrodes of the biosensor 30, the biosensor 30 outputs the response current value corresponding to the glucose concentration in the blood.

The detection apparatus 3 measures the response current value outputted from the biosensor 30 in a way that controls the voltage applied to between the electrodes of the biosensor 30. When the voltage is applied to between the electrodes of the biosensor 30, the glucose in the blood is oxidized by the glucose oxidoreductase, and the electrons being thereby extracted are supplied to the working electrode. The detection apparatus 3 measures, as the response current value, the quantity of electric charges of the electrons supplied to the working electrode. The detection apparatus 3 converts the response current value into the voltage value, and may measure, as the response voltage value, the quantity of electric charges of the electrons supplied to the working electrode. The first embodiment will discuss a case that the detection apparatus 3 measures the response current value.

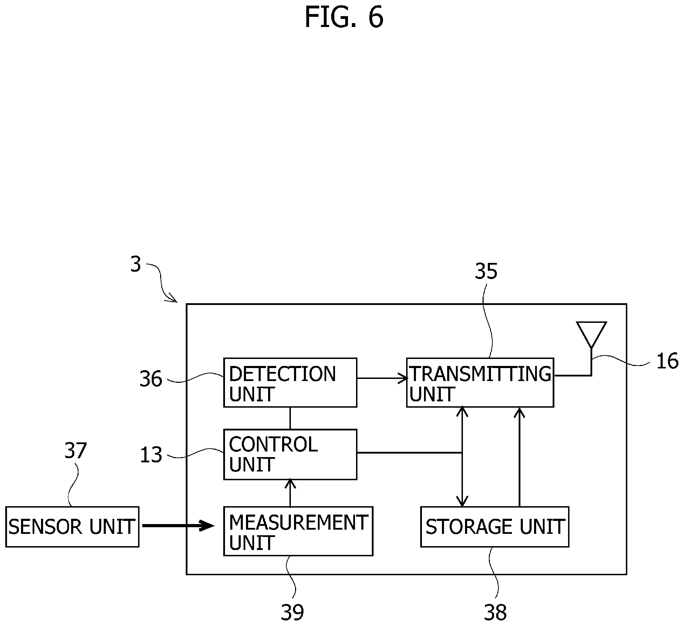

FIG. 6 is a diagram illustrating one example of a configuration of the detection apparatus 3 according to the first embodiment. In FIG. 6, the same components as those of the measuring apparatus 1 illustrated in FIG. 2 are marked with the same numerals and symbols, while their explanations are omitted. A sensor unit 37 corresponds to the biosensor 30 depicted in FIG. 5. The storage unit 38 is different from the storage unit 22 illustrated in FIG. 2 in that the storage unit 38 pre-stores the calibration curve data representing the associative relation between the response current value and the glucose concentration in the blood. A measurement unit 39 measures the glucose concentration in the blood retained in the capillary of the biosensor 30 inserted into the sensor insertion port 34. A method by which the measurement unit 39 measures the glucose concentration is the same as the method by the measurement unit 12 depicted in FIG. 2 except a point that the measurement unit 39 refers to the calibration curve data stored in the storage unit 38. A detection unit 36 detects that the operation button 32 is depressed. As triggered by an event that the detection unit 36 detects the depression of the operation button 32, a transmitting unit 35 transmits the connection request for "pairing" to the display apparatus 2 via the antenna 16. The connection request contains the identifying information of, e.g., the detection apparatus 3. The identifying information of the detection apparatus 3 is, for instance, a serial number of the detection apparatus 3. The serial number is stored in, e.g., the storage unit 38. Therefore, the control unit 13 reads the serial number as the identifying information from the storage unit 38, and transmitting unit 35 may include the serial number read by the control unit 13 in the connection request. The transmitting unit 35 transmits the response current value representing the glucose concentration measured by the measurement unit 39 to the display apparatus 2 established "pairing". The response current value transmitted from the detection apparatus 3 is used as the reference value in the display apparatus 2. The detection apparatus 3 is one example of a "detection apparatus". The response current value transmitted from the detection apparatus 3 is one example of "data for correction".

### <Processing Flow of Pairing>

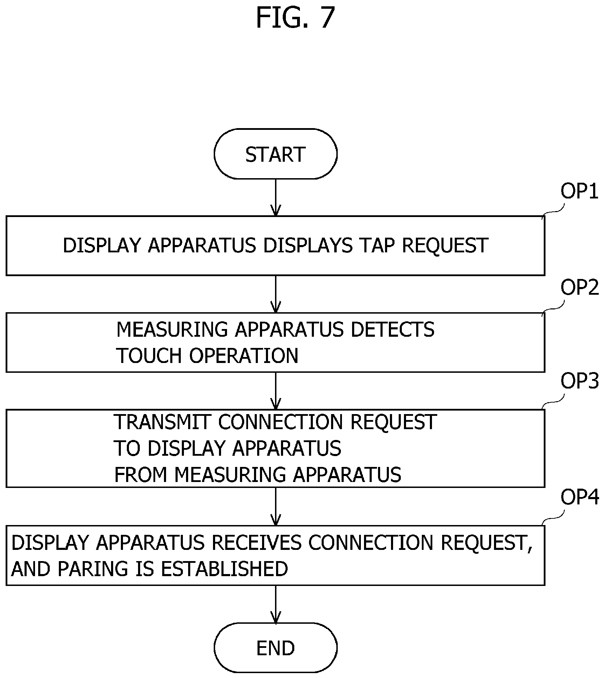

FIG. 7 is a flowchart illustrating one example of a processing flow of "pairing" according to the first embodiment. FIG. 7 illustrates "pairing" between the display apparatus 2 and the measuring apparatus 1. One example of the processing flow of "pairing" according to the first embodiment will hereinafter be described with reference to FIG. 7.

In OP1, for example, the control unit 21 of the display apparatus 2 causes the message prompting "pairing" to be displayed on the display, in response to the operation performed on the operation unit 26, and transitions to the waiting status of accepting the connection request. The message displayed on the display is, e.g., the message illustrated in FIG. 4.

In OP2, the detection unit 17 of the measuring apparatus 1 detects the touch operation done by the user. In OP3, when the detection unit 17 detects the touch operation in OP2, the transmitting unit 15a transmits, to the display apparatus 2, the connection request for the display apparatus 2 to make "pairing" via the antenna 16. The process in OP3 is one example of a process of "the transmitting unit transmits a connection request to the display apparatus when the touch sensor detects the touch operation".

In OP4, the receiving unit 23b of the display apparatus 2 receives the connection request transmitted by the measuring apparatus 1 via the antenna 24. The receiving unit 23b sends the received connection request to the control unit 21. The control unit 21 establishes "pairing" with the measuring apparatus 1 which is the transmission source of the connection request. When "pairing" is established, the display apparatus 2 is enabled to continuously receive the measurement results from the measuring apparatus 1 established "pairing".

The processes in OP1 through OP4 are repeatedly executed, thereby enabling the display apparatus 2 to establish "pairing" with the plurality of measuring apparatuses 1. In the display apparatus 2, upon receiving the response current value from the measuring apparatus 1 established "pairing", the control unit 21 refers to the calibration curve data stored in the storage unit 22, and thus converts the received response current value into the glucose concentration value. The control unit 21 displays the glucose concentration value converted from the response current value on the display of the display unit 25.

In the first embodiment, the measuring apparatus 1, upon detecting the touch operation, transmits the connection request (OP2, OP3 in FIG. 7). The display apparatus 2, when receiving the connection request in the waiting status, establishes "pairing" with the measuring apparatus 1 which is the transmission source of the connection request (OP4 in FIG. 7). According to the first embodiment, it is feasible to establish "pairing" between the measuring apparatus 1 and the display apparatus 2 by a simple operation of touching the measuring apparatus 1. The target measuring apparatus 1, established "pairing", is touched, and hence the measuring apparatus 1 is restrained from being mistaken. "Pairing" between the measuring apparatus 1 and the display apparatus 2 is established through wireless communications, and therefore the measuring apparatus 1 and the display apparatus 2 may not be made to approach or contact each other. Consequently, for instance, even when the measurement examinee is attached with the measuring apparatus 1 is in a sleeping state, it is possible to easily establish "pairing" between the measuring apparatus 1 and the display apparatus 2.

In the first embodiment, the display apparatus 2 not transitioning to the waiting status discards the received connection request. Hence, according to the first embodiment, "pairing" between the measuring apparatus 1 and the unexpected display apparatus 2 is restrained from being established.

The establishment of "pairing" enables the measuring apparatus 1 to continuously transmit the measurement results to the display apparatus 2. Therefore, according to the first embodiment, it is feasible that the measuring apparatus 1 transmits the measurement results to the display apparatus 2 in real time.

### <First Modified Example>

In the first embodiment, "pairing" between the measuring apparatus 1 and the display apparatus 2 is established as triggered by the connection request transmitted from the measuring apparatus 1. In a first modified example, "pairing" between the measuring apparatus 1 and the display apparatus 2 is established as triggered by the connection request transmitted from the display apparatus. The first modified example will hereinafter be described with reference to the drawings.

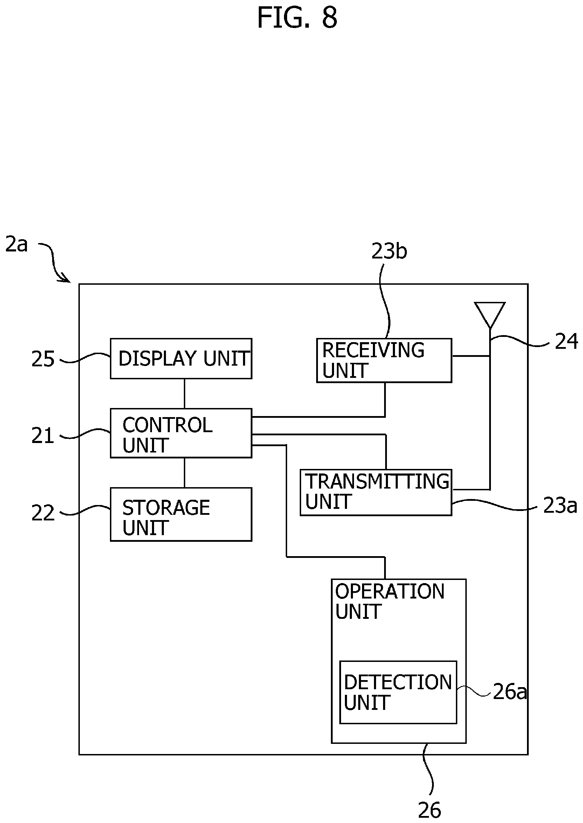

FIG. 8 is a diagram illustrating one example of a configuration of a display apparatus 2a according to the first modified example. The display apparatus 2a is different from the display apparatus 2 according to the first embodiment in that the operation unit 26 includes a detection unit 26a. The detection unit 26a is a sensor to detect the touch operation performed by the user, and is enabled to adopt various types of sensors as in the case of the detection unit 17 of the measuring apparatus 1. When the detection unit 26a detects the touch operation, coordinate values representing a position where the touch operation is conducted are transmitted to the control unit 21. In the display apparatus 2a according to the first modified example, the detection unit 26a is so disposed as to be superposed on the display of the display unit 25. The detection unit 26a is so disposed as to be superposed on the display and is thereby enabled to detect the touch operation on an icon displayed on the display. The detection unit 26a is one example of a "touch sensor".

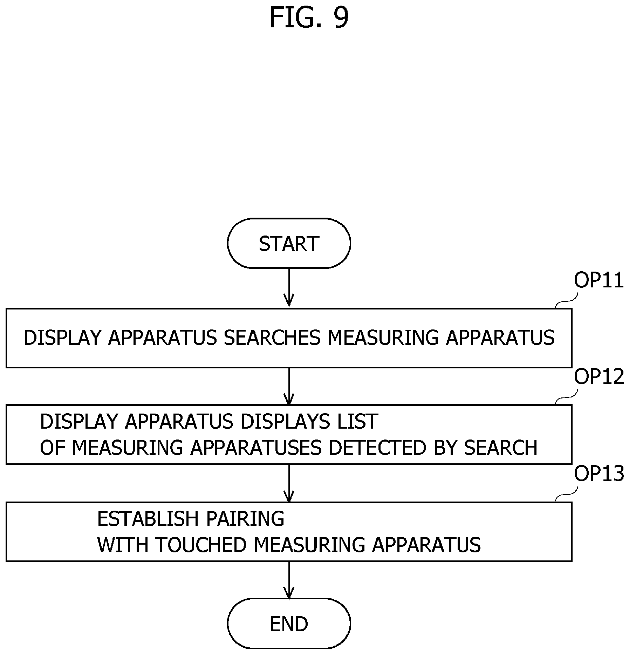

FIG. 9 is a flowchart illustrating one example of a processing flow of "pairing" according to the first modified example. One example of the processing flow of "pairing" according to the first modified example will hereinafter be described with reference to FIG. 9.

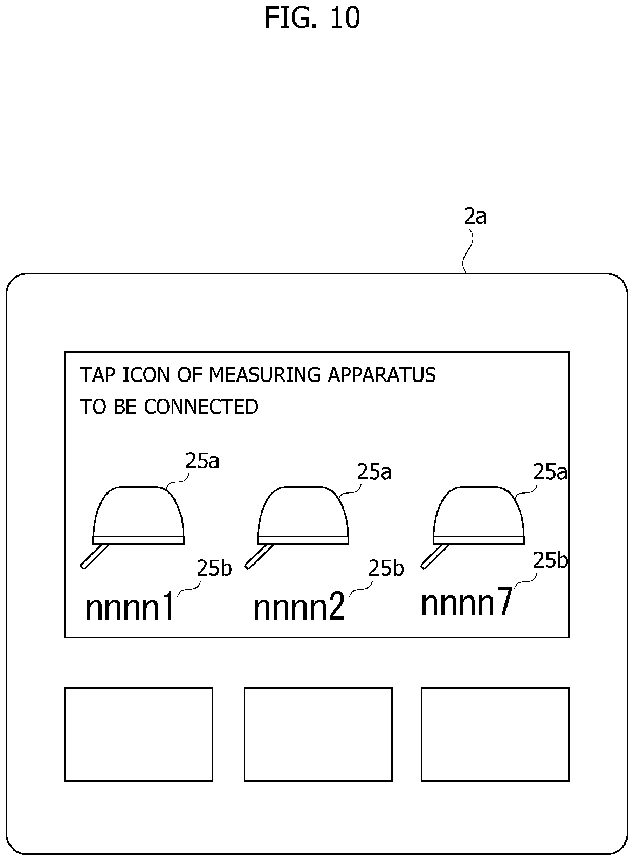

In OP11, the control unit 21 of the display apparatus 2a transmits a search command for searching the measuring apparatus 1 existing in the periphery of the display apparatus 2a. The search command is, e.g., a Bluetooth (registered trademark) Device Inquiry command. Upon receiving the search command, the control unit 13 of the measuring apparatus 1 transmits the identifying information of the measuring apparatus 1. In OP12, the display apparatus 2a displays, on the display of the display unit 25, information specifying the measuring apparatus of the identifying information received through the search in OP11. FIG. 10 is a diagram illustrating one example of the display apparatus 2a, in which the information specifying the measuring apparatus 1 is displayed on the display. In FIG. 10, pieces of information specifying the three measuring apparatuses 1 searched by the search command, are displayed on the display. In FIG. 10, an icon 25a and identifying information 25b of the measuring apparatus 1 are exemplified as the information specifying the measuring apparatus 1.

In OP13, the detection unit 26a of the display apparatus 2a detects the user's touch operation on the icon 25a. The detection unit 26a, which detects the touch operation, notifies the control unit 21 of coordinate information indicating a position where the touch operation is detected. The control unit 21 determines, based on the notified coordinate information, which icon 25a undergoes execution of the touch operation. The control unit 21 transmits the connection request to the measuring apparatus 1 specified by the touched icon 25a. "Pairing" between the display apparatus 2a and the selected measuring apparatus 1 is established owing to the process in OP13. The process in OP13 is one example of a "process of transmitting a connection request to the connection target measuring apparatus".

In the first modified example, when the plurality of measuring apparatuses 1 is provided, a list of pieces of information specifying the plurality of measuring apparatuses 1 is displayed on the display of the display unit 25 of the display apparatus 2a. The control unit 21 of the display apparatus 2a establishes "pairing" between the measuring apparatus 1 selected from the displayed list and the display apparatus 2a. Hence, according to the first embodiment, even when the plurality of measuring apparatuses 1 is provided, it is easy to establish "pairing" between the selected measuring apparatus 1 and the display apparatus 2a.

### <Second Modified Example>

In the first embodiment and the first modified example, "pairing" between the measuring apparatus 1 and the display apparatus 2 is established. In a second modified example, "pairing" between the detection apparatus 3 and the display apparatus 2 is established. The second modified example will hereinafter be described with reference to the drawings.

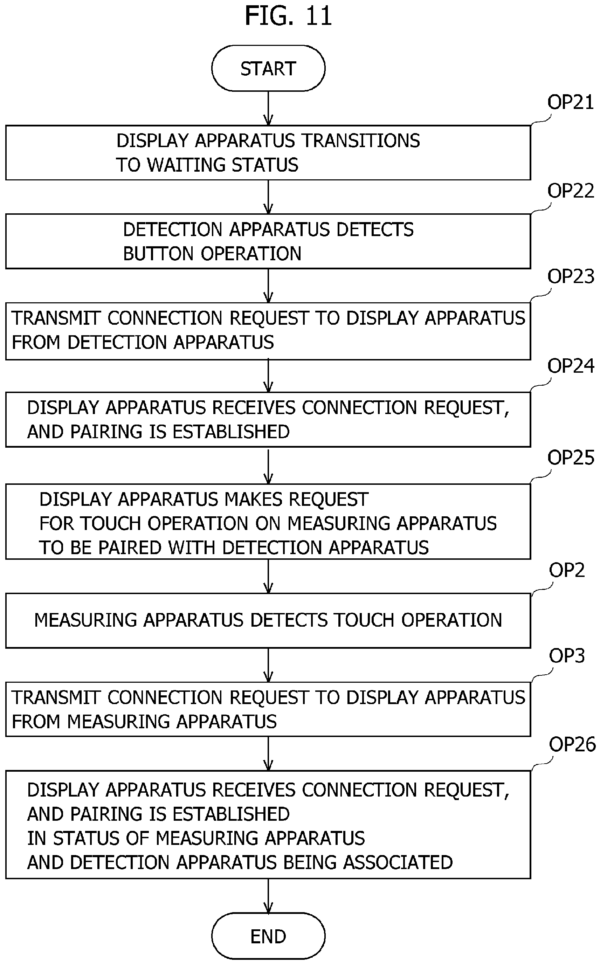

FIG. 11 is a flowchart illustrating one example of a processing flow of "pairing" according to the second modified example. The same processes as those in FIG. 7 are marked with the same numerals and symbols, while their explanations are omitted. One example of the processing flow of "pairing" according to the second modified example will hereinafter be described with reference to FIG. 11.

In OP21, the control unit 21 of the display apparatus 2 causes the message prompting "pairing" with the detection apparatus 3 to be displayed on the display, and transitions to the waiting status of accepting the connection request. For example, the message to be displayed on the display is what the "measuring apparatus" is replaced with the "detection apparatus" in the message illustrated in FIG. 4.

In OP22, the detection unit 36 of the detection apparatus 3 detects the depression of the operation button 32. In OP23, the transmitting unit 35 of the detection apparatus 3, as triggered by detecting the depression of the operation button 32 in OP22, transmits the connection request for "pairing" to the display apparatus 2 via the antenna 16.

In OP24, the receiving unit 23b of the display apparatus 2 receives the connection request from the detection apparatus 3 via the antenna 24. The receiving unit 23b sends the received connection request to the control unit 21. The control unit 21 establishes "pairing" with the detection apparatus 3 which is the transmission source of the connection request. The control unit 21, for example, when establishing "pairing", stores the identifying information contained in the connection request in the storage unit 22.

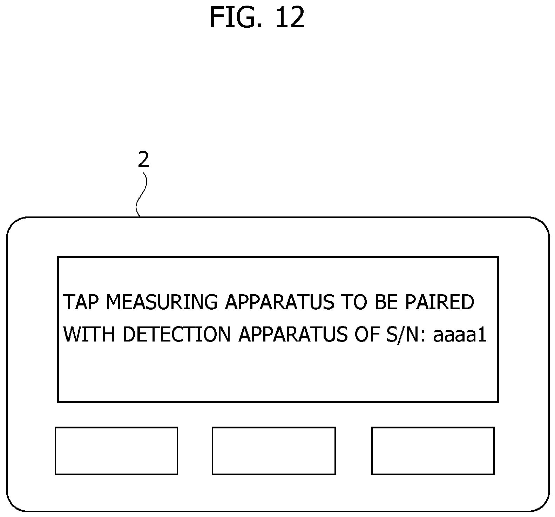

In OP25, the control unit 21 displays, on the display, the message prompting "pairing" with the measuring apparatus 1 associated with the detection apparatus 3 established "pairing". FIG. 12 is a diagram illustrating one example of the display apparatus 2 on which to display the message prompting "pairing" with the measuring apparatus 1 associated with the detection apparatus 3. A serial number of the detection apparatus 3, established "pairing" in OP24, is displayed on the display of the display apparatus 2. The processes in OP2, OP3 are the same as the processes in OP2, OP3 of FIG. 7. Consequently, their explanations are omitted.

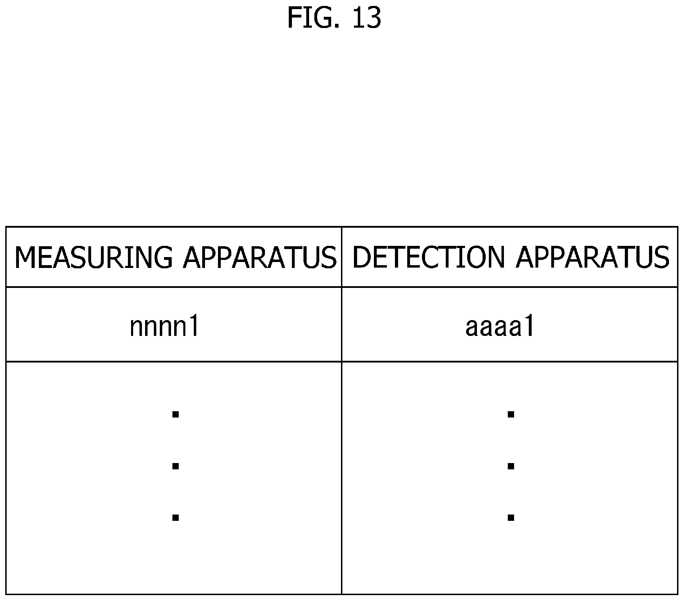

In OP26, the receiving unit 23b of the display apparatus 2 receives the connection request from the measuring apparatus 1 via the antenna 24. The receiving unit 23b sends the received connection request to the control unit 21. The control unit 21 establishes "pairing" with the measuring apparatus 1 which is the transmission source of the connection request. The control unit 21 extracts the identifying information from the received connection request. The control unit 21 associates the measuring apparatus 1 identified by the extracted identifying information with the detection apparatus 3 established "pairing" in OP24. The association is done by associating the identifying information of the measuring apparatus 1 with the identifying information of the detection apparatus 3 and storing the associated information in the storage unit 22. FIG. 13 is a diagram illustrating one example of a table configured to associate the identifying information of the measuring apparatus 1 with the identifying information of the detection apparatus 3. FIG. 13 illustrates "nnnn1" as the identifying information of the measuring apparatus 1 and "aaaa1" as the identifying information of the detection apparatus 3 associated with the measuring apparatus 1 specified by the identifying information "nnnn1". The display apparatus 2 is enabled to associate the measuring apparatus 1 with the detection apparatus 3 through the table illustrated in, e.g., FIG. 13. The process in OP26 is one example of a "process of associating a detection apparatus with the connection target measuring apparatus and storing the associated information in the storage unit, when a connection with the connection target measuring apparatus is established, the detection apparatus transmitting data for correction of data received from the connection target measuring apparatus".

In the second modified example, the display apparatus 2 is enabled to associate the measuring apparatus 1 with the detection apparatus 3 that transmits the reference value used for correcting the response current value transmitted from this measuring apparatus 1. Therefore, according to the second modified example, the response current value transmitted from the measuring apparatus 1 may be corrected by using the reference value given from the detection apparatus 3 associated with this measuring apparatus 1. In other words, according to the second modified example, the detection apparatus 3 transmitting the reference value used for correcting the response current value given from the measuring apparatus 1 is restrained from being mistaken. Note that after "pairing" between the detection apparatus 3 and the display apparatus 2 has been established, "pairing" between the measuring apparatus 1 and the display apparatus 2 is established in the second modified example. However, a sequential order of establishing "pairing" is not limited to this order. For example, "pairing" between the detection apparatus 3 and the display apparatus 2 may be established after establishing "pairing" between the measuring apparatus 1 and the display apparatus 2.

Note that "pairing" with the measuring apparatus 1 is further done in the processes of OP25 through OP26 after establishing "pairing" between the display apparatus 2 and the detection apparatus 3 in the processes of OP21 through OP24 in the second modified example; and this sequential order may, however, be replaced with each other. For example, to begin with, after "pairing" between the display apparatus 2 and the measuring apparatus 1 has been done in the processes of OP25 through OP26, "pairing" with the detection apparatus 3 may be further done in the processes of OP21 through OP24.

### <Second Embodiment

A second embodiment will be described. The following discussion will be focused on different points of the second embodiment from the first embodiment, and the same components in the second embodiment as those in the first embodiment are marked with the same numerals and symbols as those in the first embodiment, while their explanations are omitted.

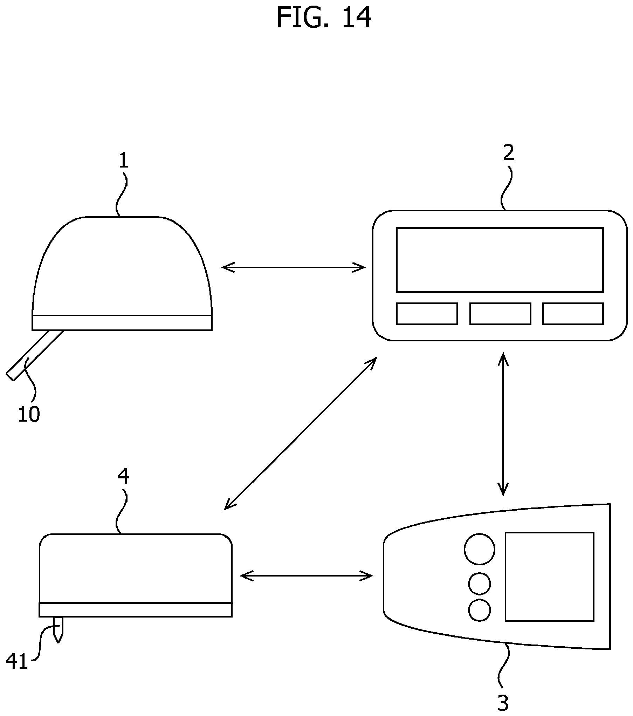

FIG. 14 is a view of a configuration of a measuring system according to the second embodiment. The measuring system illustrated in FIG. 14 includes a measuring apparatus 1, a display apparatus 2, a detection apparatus 3 and a dosage apparatus 4. The measuring apparatus 1, the display apparatus 2 and the detection apparatus 3 according to the second embodiment are the same as those in the first embodiment. The dosage apparatus 4 is a medicine supply apparatus to consecutively (continuously) or intermittently supply a medicine in vivo. The dosage apparatus 4 may be used in a state of being attached to regions instanced by an abdominal region, a brachial region and a gluteal region. The dosage apparatus 4 may take any one of a patch (paste) type and a tube type. The medicines include insulin and glucagon. The dosage apparatus 4 performs wireless data communications with the display apparatus 2, and also performs the wireless data communications with the detection apparatus 3. The dosage apparatus 4 may perform wired data communications with the display apparatus 2, and may also perform the wired data communications with the detection apparatus 3.

### <Dosage Apparatus>

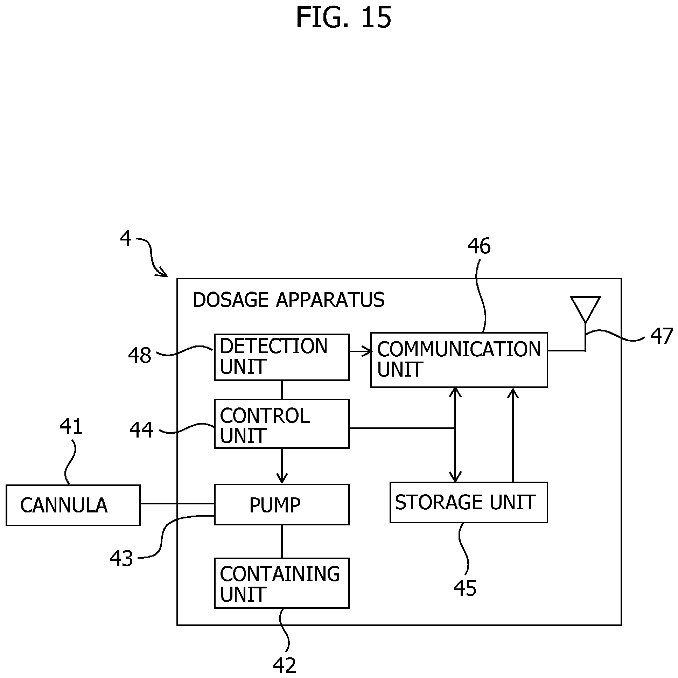

The dosage apparatus 4 includes a cannula (insertion unit) 41 used by being implanted into a subcutaneous region of the user. The dosage apparatus 4 is pasted to a skin of the user by an adhesive tape and other equivalent materials, or is attached to a piece of clothing, a belt and other equivalent articles, thereby being fitted to the user. FIG. 15 is a block diagram of a configuration of the dosage apparatus 4 according to the second embodiment. The dosage apparatus 4 includes, the cannula 41, a containing unit 42, a pump 43, a control unit (arithmetic unit) 44, a storage unit 45, a communication unit 46, an antenna 47, and a detection unit 48. The cannula 41 is connected to the pump 43. The containing unit 42 may contain plural types of medicines. A plurality of containing units 42 may also be provided in the dosage apparatus 4. For example, one unit of the plurality of containing units 42 may contain insulin, while one of the plurality of containing units 42 may also contain glucagon.

The pump 43 is actuated by, e.g., unillustrated motor. The pump 43 is actuated to feed the medicine within the containing unit 42 to the cannula 41, whereby the medicine is dosed in vivo. A plurality of pumps 43 may be provided in the dosage apparatus 4. For instance, one of the plurality of pumps 43 may be an insulin pump, while one of the plurality of pumps 43 may also be a glucagon pump. The control unit 44 controls the storage unit 45 and the communication unit 46. The control unit 44 receives various items of data from the display apparatus 2 and the detection apparatus 3 via the communication unit 46 and the antenna 47. The control unit 44 transmits the various items of data to the display apparatus 2 and the detection apparatus 3 via the communication unit 46 and the antenna 47. The control unit 44, the storage unit 45 and the communication unit 46 may be attained by: computers each including the CPU, the RAM, the ROM and other equivalent hardware components that are provided in the dosage apparatus 4; respective apparatuses; and programs and other equivalent software components running on the computer.

The detection unit 48 is a sensor that detects the touch operation of the user instanced by the measurement examinee and the medical doctor. A mechanism by which the detection unit 48 detects the touch operation is the same as by the detection unit 17 of the measuring apparatus 1 described above. The communication unit 46 transmits, as triggered by the detection unit 48 detecting the touch operation, the connection request for "pairing" to the display apparatus 2 via the antenna 47. The connection request contains, e.g., identifying information of the dosage apparatus 4. The identifying information of the dosage apparatus 4 may be a serial number of the dosage apparatus 4. The serial number is stored in, e.g., the storage unit 45. Therefore, the control unit 44 reads the serial number as the identifying information from the storage unit 45, and the communication unit 46 is enabled to include the serial number read by the control unit 44 in the connection request.

The control unit 44 acquires the glucose concentration value in the interstitial fluid from the display apparatus 2. The control unit 44 may also acquire the response current value from the display apparatus 2. The calibration curve data representing the associative relation between the response current value and the glucose concentration value in the interstitial fluid may be stored in the storage unit 45. The control unit 44 may also acquire the glucose concentration value in the interstitial fluid by converting the response current value into the glucose concentration value with reference to the calibration curve data. The control unit 44 acquires the glucose concentration value in the blood from the display apparatus 2 or the detection apparatus 3. The control unit 44 may also acquire the current value from the display apparatus 2 or the detection apparatus 3. The calibration curve data representing a relation between the current value and the glucose concentration value in the blood may be stored in the storage unit 45. The calibration curve data as, e.g., a mathematical expression and an associative table are pre-stored in the storage unit 45. The control unit 44 may also acquire the glucose concentration value in the blood by converting the current value into the glucose concentration value in the blood with reference to the calibration curve data.

The control unit 44 determines whether the pump 43 is actuated, based on the glucose concentration value in the interstitial fluid or the glucose concentration value in the blood. The following discussion will be focused on a case that the control unit 44 controls the pump 43 on the basis of the glucose concentration value in the interstitial fluid. The control unit 44 controls the pump 43 on the basis of the glucose concentration value in the blood, in which case the same control as the following control is carried out by replacing "the glucose concentration value in the interstitial fluid" in the following discussion with "the glucose concentration value in the blood".

The control unit 44 may determine whether the glucose concentration value in the interstitial fluid is equal to or larger than a predetermined value. The control unit 44 may also control the pump 43 so that the medicine is dosed in vivo when the glucose concentration value in the interstitial fluid is equal to or larger than the predetermined value. The control unit 44 may also determine whether the glucose concentration value in the interstitial fluid is less than the predetermined value. The control unit 44 may also control the pump 43 so that the medicine is dosed in vivo when the glucose concentration value in the interstitial fluid is less than the predetermined value. The control unit 44 may also determine whether the glucose concentration value in the interstitial fluid falls within a predetermined range. The control unit 44 may also control the pump 43 so that the medicine is dosed in vivo when the glucose concentration value in the interstitial fluid does not fall within the predetermined range.

The control unit 44 determines a type of medicine and a dosage (unit/min) of medicine, corresponding to the glucose concentration value in the interstitial fluid. The control unit 44 may determine, based on the glucose concentration value in the interstitial fluid, whether insulin or glucagon is dosed. The control unit 44 may transmit the determined type of medicine and the determined dosage of medicine to the display apparatus 2 via the communication unit 46. In this case, the display apparatus 2 may display the received type of medicine and the received dosage of medicine on the display unit 25.

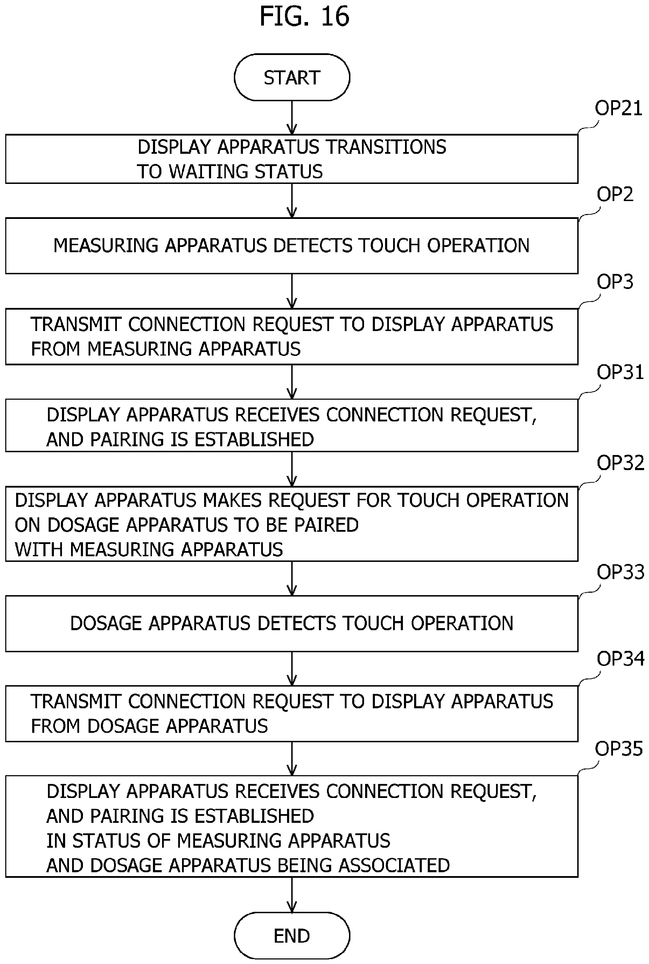

FIG. 16 is a flowchart illustrating one example of a processing flow of "pairing" according to the second embodiment. In FIG. 16, the same processes as those in FIGS. 7 and 11 are marked with the same numerals and symbols, while their explanations are omitted. One example of the processing flow of "pairing" according to the second embodiment will hereinafter be described with reference to FIG. 16.

In OP31, the receiving unit 23b of the display apparatus 2 receives the connection request from the measuring apparatus 1 via the antenna 24. The receiving unit 23b sends the received connection request to the control unit 21. The control unit 21 establishes "pairing" with the measuring apparatus 1 which is the transmission source of the connection request. The control unit 21, for example, when establishing "pairing", stores the identifying information contained in the connection request in the storage unit 22.

In OP32, the control unit 21 displays, on the display, the message prompting "pairing" with the dosage apparatus 4 to be associated with the measuring apparatus 1 established "pairing". The message to be displayed is what the "measuring apparatus" is replaced with the "dosage apparatus" in the message illustrated in, e.g., FIG. 12.

In OP33, the detection unit 48 of the dosage apparatus 4 detects the user's touch operation. In OP34, the communication unit 46, when the detection unit 48 detects the touch operation in OP33, transmits the connection request for "pairing" to the display apparatus 2 via the antenna 16.

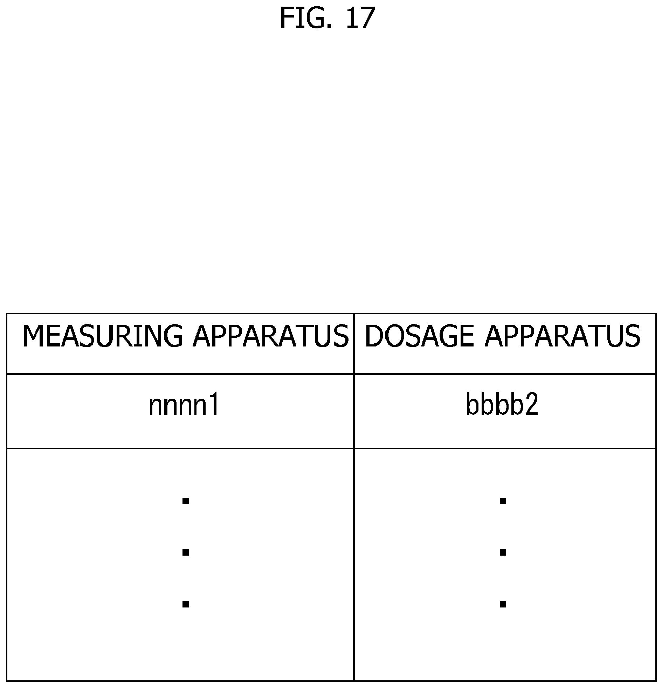

In OP35, the display apparatus 2 receives the connection request from the dosage apparatus 4 via the antenna 24, and establishes "pairing" with the dosage apparatus 4 which is the transmission source of the connection request. The control unit 21 extracts the identifying information from the received connection request. The control unit 21 associates the dosage apparatus 4 identified by the extracted identifying information with the measuring apparatus 1 established "pairing" in OP31. The association is done by associating the identifying information of the measuring apparatus 1 with the identifying information of the dosage apparatus 4 and storing the associated information in the storage unit 22. FIG. 17 is a diagram illustrating one example of a table configured to associate the identifying information of the measuring apparatus 1 with the identifying information of the dosage apparatus 4. FIG. 17 illustrates "nnnn1" as the identifying information of the measuring apparatus 1 and "bbbb2" as the identifying information of the dosage apparatus 4 associated with the measuring apparatus 1 specified by the identifying information "nnnn1". The display apparatus 2 is enabled to associate the dosage apparatus 4 with the measuring apparatus 1 through the table depicted in, e.g., FIG. 17.

The embodiments and the modified examples disclosed above can be respectively combined. For example, by combining the first modified example with the second modified example, the display apparatus 2 may transmit the connection request to the measuring apparatus 1 on the occasion of "pairing" between the display apparatus 2 and the measuring apparatus 1 in the first modified example. After associating the dosage apparatus 4 with the measuring apparatus 1 in the second embodiment, "pairing" with the detection apparatus 3 is executed, and the dosage apparatus 4, the measuring apparatus 1 and the detection apparatus 3 may be associated with each other.

## Claims

1. A measuring apparatus performing wireless communications with a display apparatus, the display apparatus displaying a measurement result of a specified substance in a body fluid, the measuring apparatus comprising:
a sensor configured to be indwelled in vivo;
a transmitting unit configured to transmit data relating to the specified substance obtained by using the sensor; and
a touch sensor configured to detect a touch operation of a user,
whrein the transmitting unit transmits a connection request to the display apparatus when the touch sensor detects the touch operation.

2. A display apparatus configured to display a measurement result of a specified substance in body fluid by using data received from a measuring apparatus, the display apparatus comprising:
a display unit configured to display a plurality of measuring apparatuses connectable for wireless communications;
a touch sensor configured to detect a touch operation indicating a connection target measuring apparatus selected from within the plurality of measuring apparatuses; and
a control unit configured to execute a process of transmitting a connection request to the connection target measuring apparatus.

3. The display apparatus according to claim 2, further comprising a storage unit,
the control unit executing a process of associating a detection apparatus with the connection target measuring apparatus and storing the associated information in the storage unit, when a connection with the connection target measuring apparatus is established, the detection apparatus transmitting data for correction of data received from the connection target measuring apparatus.

4. A display apparatus configured to display a measurement result of a specified substance in body fluid by using data received from a measuring apparatus, comprising:
a storage unit; and
a control unit configured to establish a connection with the measuring apparatus in response to a first connection request received from the measuring apparatus,
wherein the control unit executes a process of associating the measuring apparatus with a dosage apparatus in response to a second connection request received from the dosage apparatus to dose a medicine in vivo and storing the associated information in the storage unit, when a connection with the measuring apparatus is established.

5. The display apparatus according to claim 4, wherein the control unit executes a process of associating the measuring apparatus with an apparatus which is a transmission source of the data for correction of the data received from the measuring apparatus, when the connection with the measuring apparatus is established.
